# EUROPEAN PATENT APPLICATION

(11) **EP 2 491 920 A1**
(43) Date of publication of application: **29.08.2012**
(21) Application number: 11155915.9
(22) Date of filing: 25.02.2011
(51) Int. Cl.: A61K 9/16, A61K 31/47

(54) **Effervescent tablet, sachet and dry syrup of gemifloxacin**

(71) Applicant: Deva Holding Anonim Sirketi, 34303 Istanbul (TR)
(72) Inventor: Haas, Philipp Daniel, 34303, ISTANBUL (TR); Sivasligil, Ramazan, 34303, ISTANBUL (TR); Koc, Fikret, 34303, ISTANBUL (TR); Kandemir, Levent, 34303, ISTANBUL (TR)

(57) **Abstract**

The invention encompasses soluble effervescent tablet, sachet and dry syrup formulations and manufacturing methods of gemifloxacin or pharmaceutically acceptable salt.

## Description

### Technical Field

This invention encompasses effervescent tablet, sachet and dry syrup formulations of gemifloxacin or pharmaceutically acceptable salt and manufacturing methods thereof.

### Background Art

Gemifloxacin was firstly disclosed by EP 688772 B (LG CHEMICAL LTD.) 05.06.1999 .EP688772 describes a novel quinoline carboxylic acid derivative having an antibacterial activity.

Gemifloxacin is a synthetic broad-spectrum antibacterial agent that belongs to a class of antibiotics called fluoroquinolones. Gemifloxacin's activity is covering gram-negative, gram-positive bacteria and penicillin-resistant Streptococcus pneumonia where gemifloxacin shows relatively poor activity against anaerobes.

Gemifloxacin acts by inhibiting DNA synthesis through the inhibition of both DNA gyrase and topoisomerase IV enzymes which results in rapid bacterial death.

Gemifloxacin which is known as one of the important of respiratory fluoroquinolone, is found to be especially effective against respiratory tract infections such as acute exacerbation of chronic bronchitis (AECB), community acquired pneumonia (CAP) and acute bacterial sinusitis (ABS).

### Summary of invention

This invention encompasses effervescent tablet, sachet and dry syrup formulations of gemifloxacin or pharmaceutically acceptable salt and manufacturing methods thereof.

### Technical Problem

Difficulty in swallowing which is known as dysphagia is common among all age groups.

Especially elderly people, pediatrics, person with dysphagia find difficult to swallow the tablets and/or capsules and thus do not comply with prescription which results inefficient treatment or no treatment of disease.

Furthermore pills are not always acceptable to children and indeed many adults find them objectionable to take. Likewise large sized oral solid dosage form products are particularly difficult to swallow by many people and thus commercially not preferable.

High incidence of noncompliance of patient which is a major concern results in ineffective therapy.

Developed dosage forms present an alternative to large sized gemifloxacin (Factive) tablets which is in amount of 533 mg per tablet in toto. Considering tablet weight of Factive swallowing problem bind many patients.

### Solution to Problem

In the present invention, inventors invent alternative dosage forms and pharmaceutical formulations to conventional oral solid dosage forms of gemifloxacin or pharmaceutically acceptable salt thereof.

In order to enhance the patient compliance and convenience of administration in the treatment, patient friendly dosage forms and pharmaceutical formulations are conveniently presented in the suitable form of sachet, effervescent tablet and dry syrup.

Such forms of gemifloxacin or pharmaceutically acceptable salt need to be reconstituted with water or other suitable liquid before use and may be administered as fully dissolved, dispersed or suspended in a solution. Hence they are particularly designed to be administered to patients who have difficulties to swallowing capsules or tablets.

### Description of embodiments

In the first aspect of this invention, effervescent tablet formulation of gemifloxacin or pharmaceutically acceptable salt is presented.

Oral administration of the powder or tablet in solution or suspension or dispersion provides particularly several advantages over conventional oral solid dosage forms. As the drug product is already in solution at the time it is consumed, reduced localized contact of the drug in the upper gastrointestinal tract leads to less irritation and greater tolerability to patients. Buffered drug solution prevents gastric acids from interacting with the active pharmaceutical ingredient, which can be a major cause of stomach and esophageal upsets.

Advantages of presented pharmaceutical formulations of gemifloxacin or pharmaceutically acceptable salt compared with other oral dosage forms include an opportunity for formulator to improve taste. Like other antibiotics, gemifloxacin alone has an unpleasant taste which is especially not easily acceptable to children. However superior taste masking can be achieved by complementing formulations with flavours and/or sweeteners in said dosage forms.

Although effervescent tablets are generally added to an aqueous medium such as water prior to oral administration, they may be designed to disintegrate in the mouth. Most commonly effervescent tablets are resulting in the formation of a solution or a suspension and the evolution of carbon dioxide gas.

Effervescent tablet are making use of a chemical reaction between a soluble acid and an alkali metal carbonate with the help of water to make CO₂ gas, which can give a fizzy taste in the mouth.

Typically, the acid is organic or mineral acid that is safe and approved for consumption and pharmaceutical and/or nutritional purposes which provides effective and rapid effervescent disintegration upon contact with water and the alkaline effervescent compound.

The acidity for the effervescent reaction can be obtained from three main sources: acids, acid anhydrides, and acid salts.

The food acids are most commonly used since they are generally regarded as safe (GRAS), they occur in nature and are ingestible. Example of food acid includes are, but not limited to, citric acid, tartaric acid, ascorbic acid, fumaric acid, malic acid, adipic and succinic acid, acetyl salicylic acid, nicotinic acid, mixtures thereof and the like.

Citric anhydride, succinic anhyride, glutamic anhydride can be used as well.

The acid salt of the composition can be any suitable acid salt or any mixture of suitable salts. Examples of such a suitable acid salts or mixture of suitable salts includes sodium hydrogen phosphate, disodium dihydrogen pyrophospate, acid citrate salts, aminoacid hydrochlorides. Combinations thereof are possible.

In effervescent formulations, alkaline excipients can be used. The alkaline component can be any suitable alkaline effervescent compound those are safe and approved for pharmaceutical and/or nutritional purposes, and typically it is an organic base (e.g., an alkali metal carbonate). It provides an effective and rapid effervescent disintegration upon contact with water and the acid compound. The alkaline effervescing compound may be selected from the group consisting of carbonate salts, bicarbonate salts, and mixtures thereof. Example of carbonate sources , but not limited to, sodium bicarbonate, sodium carbonate, potassium bicarbonate and potassium carbonate, sodium sesquicarbonate, sodium glycine carbonate, I-lysine carbonate, arginine carbonate and mixtures thereof. Alkaline earth metal carbonates and bicarbonates can be used but alkali metal carbonates and bicarbonates are preferred.

Any conventional effervescent agent for example a mixture of a substance which on dissolution in water forms an acid reacting solution, and a pharmaceutically acceptable carbonate can be used as effervescent agent.

In effervescent tablet formulation, gemifloxacin containing effervescent tablet comprises; gemifloxacin or pharmaceutically acceptable salt thereof is in the range of from about 10 % to about 90 %, diluent is in the range of from about 1 % to about 50 %, sweetener is in the range of from about 1 % to about 20%, flavouring agent is in the range of from about 0.1% to about 10%, acidic and basic effervescent agents are in the range of from about 0.1% to about 80%, lubricant is in the range of from about 0.1% to about 2 % by weight of pharmaceutical dosage form.

Preferably effervescent formulation comprises; gemifloxacin or pharmaceutically acceptable salt thereof is in the range of from about 15 % to about 85 %, diluent is in the range of from about 2 % to about 40 %, sweetener is in the range of from about 2 % to about 15%, flavouring agent is in the range of from about 0.3% to about 5%, acidic and basic effervescent agents are in the range of from about 0.3 % to about 50%, lubricant is in the range of from about 0.3 % to about 1.5 % by weight of pharmaceutical dosage form.

Most preferably, gemifloxacin effervescent tablet composition comprising gemifloxacin is 26.60%, diluent is 3.40 %, sweetener is 4.33%, flavouring agent is 2.00 % ,acidic effervescent agent is 22.67% ,basic effervescent agent is 40.00% , lubricant is 1.00% by weight of total pharmaceutical dosage form.

For effervescent tablet formulation of the present invention, gemifloxacin: diluent ratio is selected from 1:30 to 30:1 by weight. More preferably ratio is selected from 1:20 to 20:1 and most preferably from 1:3 to 10: 1.

Precisely, in gemifloxacin effervescent tablet formulation, gemifloxacin: diluents ratio is 7.82.

For effervescent tablet formulation of the present invention, gemifloxacin: basic effervescent agent ratio is selected from 1:10 to 10:1 1 by weight. More preferably ratio is selected from 1:4 to 4:1 and most preferably from 1:2 to 2:1.

Most preferably in gemifloxacin effervescent tablet formulation, gemifloxacin: basic effervescent agent ratio is 0.665.

For effervescent tablet formulation of the present invention, gemifloxacin: acidic effervescent agent ratio is selected from 1:8 to 8:1 by weight. More preferably ratio is selected from 1:2 to 2:1 and most preferably from 1:1 to 3:2.

Precisely, in gemifloxacin effervescent tablet formulation, gemifloxacin: acidic effervescent agent ratio is 1.17.

For effervescent tablet formulation of the present invention, gemifloxacin: patient compliance improvizing agents ratio is selected from 1:10 to 10:1 1 by weight. More preferably ratio is selected from 1:6 to 6:1 and most preferably from 1:5 to 5:1.

Precisely, in gemifloxacin effervescent tablet formulation, gemifloxacin: patient compliance improvizing agents ratio is 4.20.

Patient compliance improvizing agents can be from artificial or natural origin or mixtures thereof. Their function may vary such as sweeteners, flavour enhancers, taste masking agents etc.

For effervescent tablet formulation of the present invention, gemifloxacin: lubricant ratio is selected from 1:100 to 100:1 1 by weight. More preferably ratio is selected from 1:70 to 70:1 and most preferably from 1:30 to 30: 1.

Precisely, in gemifloxacin effervescent tablet formulation, gemifloxacin: lubricant ratio is 26.60.

In another aspect of this invention, inventors provide a pharmaceutical formulation and a manufacturing process for the preparation a unit dose sachet comprising gemifloxacin or pharmaceutically acceptable salt thereof and an excipient or mixtures of excipients.

As used a sachet is a single dose of a pharmaceutical formulation. The formulation is preferably in the form of a particulate material, sphere, pellets, particle or preferably granulate.

Alternatively, powder, granulate, pellets, spheres, particles of sachet may be in effervescent form or mixtures thereof. The sachet can be formulated as a powder, granulate, pellets, spheres, particles or effervescent powder, effervescent granulate, effervescent pellets, effervescent spheres, effervescent particles or mixtures.

Presented powder, granulate, pellets, spheres, particles or their mixtures of sachet may be formulated for reconstitution with water or another suitable liquid. Said powder, granulate, pellets, spheres, particles or their mixtures may be ready to use sachet's formulation which can be consumed directly from the packet.

For the purposes of the present invention a "sachet" is referred herein to any suitable single serving pack, a container, a package, an envelope or a bag and the like. Preferably the sachet is sealed and disposable.

The sachet may be made up from any suitable material, such as plastic, metal foil, paper, and combination thereof. The sachet includes a perforated region or a nick in the edge of the sachet for ease of tearing.

The sachet may be launched and sold individually or may be launched or sold in pack of two or more sachets.

In the sachet formulation, gemifloxacin containing sachet comprises; gemifloxacin or pharmaceutically acceptable salt thereof is in the range of from about 10 % to about 90 %, diluent is in the range of from about 1 % to about 50 %, sweetener is in the range of from about 1 % to about 20%, flavouring agent is in the range of from about 0.1% to about 10% by weight of pharmaceutical dosage form.

Preferably sachet comprises; gemifloxacin or pharmaceutically acceptable salt thereof is in the range of from about 15 % to about 85 %, diluent is in the range of from about 2 % to about 40 %, sweetener is in the range of from about 2 % to about 15%, flavouring agent is in the range of from about 0.3% to about 5% by weight of pharmaceutical dosage form.

Most preferably, gemifloxacin sachet composition comprising gemifloxacin is 72.54%, sweetener is 13.63%, diluent is 10.19 % and flavouring agent is 3.64 % by weight of total pharmaceutical dosage form.

For sachet formulation of the present invention, gemifloxacin: diluent ratio is selected from 1:1 to 20:1 by weight. More preferably ratio is selected from 3:1 to 15:1 and most preferably from 4:1 to 6:1.

Precisely, in gemifloxacin sachet formulation, gemifloxacin: diluent ratio is 5.32.

For sachet formulation of the present invention, gemifloxacin: patient compliance improvizing agents ratio is selected from 1:1 to 20:1 by weight. More preferably ratio is selected from 3:1 to 15:1 and most preferably from 4:1 to 6:1.

Precisely, in gemifloxacin sachet formulation, gemifloxacin: patient compliance improvizing agents ratio is 5.24.

Patient compliance improvizing agents can be from artificial or natural origin or mixtures thereof. Their function may vary such as sweeteners, flavour enhancers, taste masking agents etc.

In another aspect of this invention, gemifloxacin or pharmaceutically acceptable salts thereof are provided in so called "dry syrup" formulation.

A dry syrup preparation means "preparation with water which is dissolved or suspended before use". The powder is provided in a suitable container such as glass or polyethylene bottle to which prior to use prescribed amount of water is added to give dispersion. The desired amount of the dispersion is taken orally by the patient according to his or her need. Such multiple variable "dry syrup" can be particularly used for children.

In dry syrup formulation, gemifloxacin containing dry syrup comprises; gemifloxacin or pharmaceutically acceptable salt thereof is in the range of from about 10 % to about 90%, viscosity increasing agent is in the range of from about 1% to about 20%, buffering agents is in the range of from about 0.1% to about 2 %, sweetener is in the range of from about 1 % to about 20%, suspending agents are in the range of from about 1 % to about 25%, diluent is in the range of from about 1 % to about 80 %, flavouring agent is in the range of from about 0.1% to about 10% by weight of pharmaceutical dosage form.

Preferably dry syrup formulation comprises; gemifloxacin or pharmaceutically acceptable salt thereof is in the range of from about 15 % to about 85 %,viscosity increasing agent is in the range of from about 3% to about 15 %, buffering agents is in the range of from about 0.3 % to about 1.5 % ,sweetener is in the range of from about 2 % to about 15%, suspending agents are in the range of from about 2 % to about 18%, diluent is in the range of from about 2 % to about 70 %, flavouring agent is in the range of from about 0.3% to about 5% by weight of pharmaceutical dosage form.

Most preferably, gemifloxacin dry syrup composition comprising gemifloxacin is 18.62%, viscosity increasing agent is 10.53 %, buffering agents is 1.06%, sweetener is 2.67% ,suspending agent is 22.67% ,diluent is 64.19% , flavouring agent is 0.73% by weight of total pharmaceutical dosage form.

For dry syrup formulation of the present invention, gemifloxacin: patient compliance improvizing agents ratio is selected from 1:10 to 10:1 by weight. More preferably ratio is selected from 1:6 to 8:1 and most preferably from 1:4 to 4:1.

Most preferably in gemifloxacin dry syrup formulation, gemifloxacin patient compliance improvizing agents ratio is 3.4.

Patient compliance improvizing agents can be from artificial or natural origin or mixtures thereof. Their function may vary such as sweeteners, flavour enhancers, taste masking agents etc.

For dry syrup formulation of the present invention, gemifloxacin: suspending agent ratio is selected from 1:10 to 10:1 1 by weight. More preferably ratio is selected from 1:6 to 8:1 and most preferably from1:4 to 4:1.

Most preferably in gemifloxacin dry syrup formulation, gemifloxacin: suspending agent ratio is 0.82.

For dry syrup formulation of the present invention, gemifloxacin: viscosity increasing agent ratio is selected from 1:10 to 10:1 1 by weight. More preferably ratio is selected from 1:7 to 7:1 and most preferably from 1:3 to 3:1.

Most preferably in gemifloxacin dry syrup formulation, gemifloxacin: viscosity increasing agent ratio is 1.77.

For dry syrup formulation of the present invention, gemifloxacin: diluent ratio is selected from 1:8 to 8:1 by weight. More preferably ratio is selected from 1:5 to 5:1 and most preferably from 1:5 to 4: 1.

Most preferably in gemifloxacin dry syrup formulation, gemifloxacin: diluent ratio is 0.29.

For dry syrup formulation of the present invention, gemifloxacin: buffering agents ratio is selected from 1:200 to 1:2 by weight. More preferably ratio is selected from 1: 150 to 1:10 and most preferably from 1:100 to 1: 15.

Most preferably in gemifloxacin dry syrup formulation, gemifloxacin: buffering agent ratio is 0.011.

The present invention's formulation comprises pharmaceutically acceptable excipients such as diluents, binders, disintegrants and lubricants, glidants etc.

Diluents are inert bulking agents which are added to active pharmaceutical ingredient to make a reasonably sized tablet. Diluents are, but not limited to, anhydrous lactose, lactose monohydrate, modified lactose, dibasic calcium phosphate, tribasic calcium phosphate, microcrystalline cellulose, silicified microcrystalline cellulose, powdered cellulose, maize starch, pregelatinized starch, calcium carbonate, sucrose, glucose, dextrates, dextrins, dextrose, fructose, kaolin, lactitol, maltose, mannitol, sorbitol, starch, mixtures thereof and the like.

Binders are agents used to impart cohesive qualities to the powdered material. Binders impart a cohesiveness to the tablet formulation that ensures that the tablet remain intact after compression. Tablet binders used in the mixture include tablet binders commonly used for tablet preparation. Binders are, but not limited to, polyvinylpyrolidone, copovidone, starches such as pregelatinized starch or plain starch, cellulose derivatives such as hydroxypropylmethylcellulose, ethylcellulose, hydroxypropylcellulose and carboxymethylcellulose and their salts, gelatine, acacia, agar, alginic acid,carbomer, ceratonia, chitosan, dextrates,dextrin, glycerol dibehenate, guar gum, hypromellose, inulin, magnesium aluminumsilicate, maltodextrin, poloxamer, polycarbophil, polydextrose, polyethylene oxide, polymethacrylates, sodium alginate, sucrose, hydrogenated vegetable oil, mixtures thereof and the like.

Disintegrants are, but not limited to, modified starches, croscarmallose sodium, carboxymethylcellulose calcium, sodium starch glycolate, crospovidone, alginic acid, calcium alginate, microcrystalline cellulose, powdered cellulose, chitosan, colloidal silicon dioxide, crospovidone, guar gum, low-substituted hydroxypropylcellulose, hydroxypropyl starch, magnesium aluminium silicate, methylcellulose, polacrilin potassium, sodium alginate, starch, pregelatinized starch, mixtures thereof and the like.

Lubricants prevent adhesion of the tablet material to equipment, reduce interparticle friction, and facilitate the ejection of the tablet from the die cavity. Tablet lubricants added to the milled dried granulate include those typically used in tablet formulations. Lubricants are, but not limited to, magnesium stearate, calcium stearate, hydrogenated castor oil, glyceryl behenate, glyceryl monostearate, glyceryl palmitostearate, leucine, mineral oil, light mineral oil, myristic acid, palmitic acid, polyethylene glycol, potassium benzoate, sodium benzoate, sodium lauryl sulfate, sodium stearyl fumarate, stearic acid, talc, hydrogenated vegetable oil, zinc stearate, magnesium lauryl sulphate, sodium stearyl fumarate, polyethylene glycol, stearic acid, colloidal silicon dioxide or mixtures thereof and the like.

The compositions of the present invention preferably also may contain a glidant. Glidants are preferably selected from the group consisting of colloidal silicon dioxide, precipitated silica and pyrogenic silica, talc and aluminium silicate, tribasic calcium phosphate, calcium stearate, powdered cellulose, magnesium oxide, magnesium silicate, magnesium trisilicate, starch, talc, mixtures thereof and the like.

Pharmaceutically acceptable additives other than those described above may be included in formulations of the present invention of gemifloxacin or pharmaceutically acceptable salts.

The compositions may include flavour enhancers, preservatives, antiadherants, flavourings, colouring agents, surfactants, solubilizers, wetting agents and other excipients of common use. Additives ingredients will vary according to the type of compositions being manufactured and can be included in the pharmaceutical composition in any amount.

The term gemifloxacin or a pharmaceutically acceptable salt thereof herein encompasses that the product can be both in the anhydrous or hydrate form. Gemifloxacin is commercially available as mesylate salt.

The formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods. Direct compression and wet granulation in fluid bed granulator are preferred.

Fluid bed granulator may optionally have dehumidification system in order to reduce humidity of fluidized inlet air. Dehumidification may be achieved by utilizing available techniques, such as, but not limited to, silica gel dehumidification or chilled water dehumidification.

### Example 1: Gemifloxacin Effervescent Tablet Unit Formula

**Table 1**

| | **mg/tablet** | **% (w/w)** |
|---|---|---|
| **Ingredients** | | |
| Gemifloxacin | 398.98 | 26.60 |
| Potassium Bicarbonate | 600.00 | 40.00 |
| Citric Acid Anhydrous | 340.00 | 22.67 |
| Sucralose | 35.00 | 2.33 |
| Sodium Saccharin | 30.00 | 2.00 |
| Maltodextrin | 51.02 | 3.40 |
| Cherry Flavour | 30.00 | 2.00 |
| Sodium Stearyl Fumarate | 15.00 | 1.00 |
| **Total Tablet Weight** | **1500.00** | **100.00** |

### Example 2: Gemifloxacin Effervescent Tablet Manufacturing Method of Example 1

Pharmaceutical formulation may be prepared using methods as below without limiting scope of this invention;

1. Sweeteners (sucralose, sodium saccharin), diluent (maltodextrin) and flavour (cherry flavour) are mixed.

2. Gemifloxacin or pharmaceutically acceptable salt thereof and powder mixture in step (1) is mixed.

3. Basic effervescent agent (potassium bicarbonate) and acidic effervescent agent (citric acid anhydrous) are added to powder mixture in step (2) and mixed.

4. Optionally, powder mixture in step (3) is sieved through suitable sized sieve.

5. Lubricant (sodium stearyl fumarate) is added and mixed.

6. Effervescent tablets are compressed with suitable punches having average target weight 1500 mg/tablet.

### Example 3: Gemifloxacin Sachet Unit Formula

**Table 2**

| | **mg/sachet** | **% (w/w)** |
|---|---|---|
| **Ingredients** | | |
| Gemifloxacin | 398.98 | 72.54 |
| Sucralose | 45.00 | 8.18 |
| Saccharin | 30.00 | 5.45 |
| Maltodextrin | 56.02 | 10.19 |
| Lemon Flavour | 20.00 | 3.64 |
| **Total Tablet Weight** | **550.00** | **100.00** |

### Example 4: Gemifloxacin Sachet Manufacturing Method of Example 3

Pharmaceutical formulation may be prepared using methods as below without limiting scope of this invention;

1. Gemifloxacin or pharmaceutically acceptable salt thereof and diluent (maltodextrin) are mixed.

2. Sweeteners (sucralose and saccharin) are dissolved in alcohol.

3. Powder mixture in step (1) is granulated with solution in step (2) using fluid bed granulator.

4. Drying is continued until loss on drying is less than 1.5% (w/w).

5. Flavour (lemon flavour) is diluted geometrically with dry granules to obtain homogeneous mixture.

6. Diluted flavour (lemon flavour) is added to remaining dry granules and mixed.

7. Mixture in step (6) is filled into suitable packaging material, such as sachets, having average target powder weight of 550 mg per package.

### Example 5: Gemifloxacin Dry Syrup Unit Formula

**Table 3**

| | **mg/bottle** | **% (w/w)** |
|---|---|---|
| **Ingredients** | | |
| Gemifloxacin* | 2,792.86 | 18.62 |
| Xanthan Gum | 30.00 | 0.20 |
| Citric Acid Anhydrous | 65.00 | 0.43 |
| Sodium Citrate | 95.00 | 0.63 |
| Sucralose | 220.00 | 1.47 |
| Acesulfame Potassium | 180.00 | 1.20 |
| Colloidal Silicon Dioxide | 330.00 | 2.20 |
| Carmellose Calcium | 1550.00 | 10.33 |
| Maltitol | 9,627.14 | 64.19 |
| Orange Flavour | 110.00 | 0.73 |
| **Total Weight in Bottle** | **15,000.00** | **100.00** |

* Total quantity is equal to dosing 320 mg Gemifloxacin/day for one week treatment.

### Example 6: Gemifloxacin Dry Syrup Manufacturing Method of Example 5

Pharmaceutical formulation may be prepared using methods as below without limiting scope of this invention;

1. Viscosity increasing agents (xanthan gum, carmellose calcium), buffering agents (citric acid anhydrous, sodium citrate), sweeteners (sucralose, acesulfame potassium), suspending agent (colloidal silicon dioxide) and flavour (orange flavour) are mixed.

2. Powder mixture in step (1) is optionally sieved through a suitable sieve and mixed again.

3. Gemifloxacin or pharmaceutically acceptable salt thereof is added to powder mixture in step (2) and mixed.

4. Diluent (maltitol) and powder mixture in step (3) are added and mixed.

5. Powder mixture in step (4) is filled into suitable packaging material, such as Type III glass bottles or HDPE bottles, having average target powder weight of 15,000 mg/bottle.

## Claims

1. An effervescent tablet formulation comprising gemifloxacin or pharmaceutically acceptable salt thereof and an excipient or mixtures of excipients.

2. Effervescent tablet, as claimed in claim 1, is administered as a solution in which drug is suspended or dispersed or dissolved in said solution.

3. The effervescent tablet formulation of gemifloxacin, as claimed in claim 1, comprises: gemifloxacin or pharmaceutically acceptable salt thereof is in the range of from 10 % to 90 %, diluent is in the range of from 1 % to 50 %, sweetener is in the range of from 1 % to 20%, flavouring agent is in the range of from 0.1% to 10%, acidic and basic effervescent agents are in the range of from 0.1% to 80%, lubricant is in the range of from 0.1% to 2 % by weight of pharmaceutical dosage form, **more preferably** gemifloxacin or pharmaceutically acceptable salt thereof is in the range of from 15 % to 85 %, diluent is in the range of from 2 % to 40 %, sweetener is in the range of from 2 % to 15%, flavouring agent is in the range of from 0.3% to 5%, acidic and basic effervescent agents are in the range of from 0.3 % to 50%, lubricant is in the range of from 0.3 % to 1.5 % by weight of pharmaceutical dosage form, **most preferably** gemifloxacin is 26.60%, diluent is 3.40 %, sweetener is 4.33%, flavouring agent is 2 %, acidic effervescent agent is 22.67%, basic effervescent agent is 40%, lubricant is 1% by weight of total pharmaceutical dosage form.

4. The effervescent tablet formulation of gemifloxacin, as claimed in claim 1, comprises **a)** gemifloxacin: diluent ratio is from 1:30 to 30:1 by weight, **more preferably** ratio is from 1:20 to 20:1, **most preferably** ratio is from 1:3 to 10:1, **precisely** ratio is 7.82 **and/or, b)** gemifloxacin: basic effervescent agent ratio is from 1:10 to 10:1 1 by weight, **more preferably** ratio is from 1:4 to 4:1, **most preferably ratio is** from 1:2 to 2:1, **precisely** ratio is 0.665 **and/or, c)** gemifloxacin: acidic effervescent agent ratio is from 1:8 to 8:1 by weight, **more preferably** ratio is from 1:2 to 2:1, **most preferably** ratio is from 1:1 to 3:2, precisely ratio is 1.17 **and/or, d)** gemifloxacin: patient compliance improvizing agents ratio is from 1:10 to 10:1 by weight, **more preferably** ratio is from 1:6 to 6:1, **most preferably** ratio is from 1:5 to 5:1, **precisely** ratio is 4.20 **and/or, e)** gemifloxacin: lubricant ratio is from 1:100 to 100:1 1 by weight, **more preferably** ratio is from 1:70 to 70:1, **most preferably** ratio is from 1:30 to 30:1, **precisely** ratio is 26.60.

5. The effervescent tablet manufacturing method, as claimed in claim 1, comprises following steps; a) Sweeteners, diluent and flavour are mixed. b) Gemifloxacin or pharmaceutically acceptable salt thereof and powder mixture in step (a) are mixed. c) Basic and acidic effervescent agent are added to powder mixture in step (b) and mixed. d) Optionally, powder mixture in step (c) is sieved through suitable sized sieve and mixed. e) Lubricant is added and mixed. f) Effervescent tablets are compressed with suitable punches.

6. A unit dose of sachet containing pharmaceutical formulation comprising gemifloxacin or pharmaceutically acceptable salt thereof and an excipient or mixtures of excipients.

7. A sachet formulation, as claimed in claim 6, includes powder, granulate, pellets, spheres, particles or effervescent powder, effervescent granulate, effervescent pellets, effervescent spheres, effervescent particles or mixtures thereof.

8. The sachet formulation, as claimed in claim 6, comprises; gemifloxacin or pharmaceutically acceptable salt thereof is in the range of from 10 % to 90 %, diluent is in the range of from 1 % to 50 %, sweetener is in the range of from 1 % to 20%, flavouring agent is in the range of from 0.1% to 10% by weight of pharmaceutical dosage form, **more preferably** gemifloxacin or pharmaceutically acceptable salt thereof is in the range of from 15 % to 85 %, diluent is in the range of from 2 % to 40 %, sweetener is in the range of from 2 % to 15%, flavouring agent is in the range of from 0.3% to 5% by weight of pharmaceutical dosage form, **most preferably** gemifloxacin or pharmaceutically acceptable salt thereof is 72.54%, sweetener is 13.63%, diluent is 10.19 % and flavouring agent is 3.64 % by weight of total pharmaceutical dosage form.

9. The sachet formulation of gemifloxacin, as claimed in claim 6, comprises **a)** gemifloxacin: diluent ratio is from 1:1 to 20:1 by weight, **more preferably** ratio is from 3:1 to 15:1, **most preferably** ratio is from 4:1 to 6:1, **precisely** gemifloxacin: diluents ratio is 5.32 **and/or, b)** gemifloxacin: patient compliance improvizing agents ratio is of 1:1 to 20:1 by weight, **more preferably** ratio is from 3:1 to 15:1, **most preferably** ratio is from 4:1 to 6:1, **precisely** gemifloxacin: patient compliance improvizing agents ratio is 5.24.

10. The sachet, as claimed in claim 6, is manufactured by a method that comprises following steps; a) Gemifloxacin or pharmaceutically acceptable salt thereof and diluent are mixed. b) Sweeteners are dissolved in alcohol. c) Powder mixture in step (a) is granulated with solution in step (b) using fluid bed granulator. d) Drying is continued until target loss on drying e) Flavour is diluted geometrically with dry granules to obtain homogeneous mixture. f) Diluted flavour is added to remaining dry granules and mixed. g) Mixture in step (f) is filled into sachets.

11. A dry syrup formulation comprising gemifloxacin or pharmaceutically acceptable salt thereof and an excipient or mixtures of excipients.

12. Dry syrup formulation, as claimed in claim 11, comprises dry powder or granules or pellets or mixtures thereof.

13. The dry syrup formulation of gemifloxacin, as claimed in claim 11, comprising: gemifloxacin or pharmaceutically acceptable salt thereof is in the range of from 10 % to 90 %, viscosity increasing agent is in the range of from 1% to 20 %, buffering agents is in the range of from 0.1% to 2 %, sweetener is in the range of from 1 % to 20%, suspending agent are in the range of from 1 % to 25%, diluent is in the range of from 1 % to 80 %, flavouring agent is in the range of from 0.1% to 10% by weight of pharmaceutical dosage form, more preferably gemifloxacin or pharmaceutically acceptable salt thereof is in the range of from 15 % to 85 %, viscosity increasing agent is in the range of from 3% to 15 %, buffering agents is in the range of from 0.3 % to 1.5 %, sweetener is in the range of from 2 % to 15%, suspending agent is in the range of from 2 % to 18%, diluent is in the range of from 2 % to 70 %, flavouring agent is in the range of from 0.3% to 5% by weight of pharmaceutical dosage form, **most preferably** gemifloxacin is 18.62%, viscosity increasing agent is 10.53 %, buffering agents is 1.06%, sweetener is 2.67%, suspending agent is 22.67%, diluent is 64.19% , flavouring agent is 0.73% by weight of total pharmaceutical dosage form.

14. The dry syrup formulation of gemifloxacin, as claimed in claim 11, comprises; **a)** gemifloxacin: patient compliance improvizing agent ratio is from 1:10 to 10:1 1 by weight, **more preferably** ratio is from 1:6 to 8:1, **most preferably** ratio is from1:4 to 4:1, **precisely** ratio is 3.4 **and/or, b)** gemifloxacin: suspending agent ratio is from 1:10 to 10:1 1 by weight, more preferably ratio is from 1:6 to 8:1, most preferably ratio is from1:4 to 4:1, precisely ratio is 0.82 **and/or, c)** gemifloxacin: viscosity increasing agent ratio is from 1:10 to 10:1 1 by weight, **more preferably** ratio is from 1:7 to 7:1, **most preferably** ratio is from 1:3 to 3:1, precisely ratio is 1.77 **and/or, d)** gemifloxacin: diluent ratio is of 1:8 to 8:1 by weight, **more preferably** ratio is from 1:5 to 5:1, most preferably ratio is from 1:5 to 4:1, precisely ratio is 0.29 **and/or, e)** gemifloxacin: buffering agents ratio is from 1:200 to 1:2 by weight, more preferably ratio is from 1:150 to 1:10, **most preferably** ratio is from 1:100 to 1:15, **precisely** ratio is 0.011.

15. The dry syrup, as claimed in claim 11, is manufactured by a method that comprises following steps: a) viscosity increasing agents, buffering agents, sweeteners, suspending agent and flavour are mixed, b) powder mixture in step (a) is optionally sieved through a suitable sieve and mixed again, c) gemifloxacin or pharmaceutically acceptable salt thereof is added to powder mixture in step (b) and mixed, d) diluent and powder mixture in step (c) are added and mixed, e) powder mixture in step (d) is filled into suitable packaging material, such as Type III glass bottles or HDPE bottles.
